# EUROPEAN PATENT SPECIFICATION

(11) **EP 4 183 445 B1**
(45) Date of publication and mention of the grant of the patent: **09.04.2025**
(21) Application number: 22201004.3
(22) Date of filing: 12.10.2022
(51) Int. Cl.: A61N 1/362, A61B 5/363, A61N 1/365, A61N 1/39

(54) **METHOD AND DEVICE FOR VENTRICULAR TACHYCARDIA POLYMORPHIC\MONOMORPHIC DISCRIMINATOR**
VERFAHREN UND VORRICHTUNG FÜR POLYMORPHEN/MONOMORPHEN DISKRIMINATOR VON VENTRIKULÄRER TACHYKARDIE
PROCÉDÉ ET DISPOSITIF POUR DISCRIMINATEUR POLYMORPHE OU MONOMORPHE DE TACHYCARDIE VENTRICULAIRE

(30) Priority: 22.11.2021 US 202163281754 P; 06.10.2022 US 202217960894
(43) Date of publication of application: 24.05.2023
(73) Proprietor: Pacesetter, Inc., Sylmar, CA 91342 (US)
(72) Inventor: MANGUAL-SOTO, Jan O, Sylmar, 91342 (US); RICHER, Louis-Philippe, Sylmar, 91342 (US); DAWOUD, Fady, Sylmar, 91342 (US); POLITIS, Nikolaos, Sylmar, 91342 (US)
(74) Representative: Barker Brettell Sweden AB

(56) References cited:
- US-A- 5 193 535
- US-A- 5 447 519
- US-A1- 2007 142 736
- US-B2- 7 130 677

## Description

### TECHNICAL FIELD

Embodiments of the present disclosure generally relate to methods and devices for designate beats to correspond to be a polymorphic ventricular tachycardia (PVT) or a monomorphic ventricular tachycardia (MVT).

### BACKGROUND

Implantable cardioverter defibrillators (ICD) are designed to delivery therapy during life threatening ventricular arrhythmias such as a ventricular tachycardia (VT) or ventricular fibrillation (VF). Despite the life-saving benefits of ICDs, it has been suggested that delivery of high voltage (HV) shocks may affect mortality and quality of life in ICD patients. Risk of adverse clinical outcomes could be mitigated by avoiding therapy delivery, for instance when a VT can be allowed to self-terminate or can be terminated with anti-tachycardia pacing (ATP) therapy. This has led to a shift in ICD therapy programming, away from more aggressive slower detection rate cutoffs, shorter detection times, and rapid treatment of any VA by delivery of HV therapy. Instead, now ICDs are being programmed with more conservative faster detection rate cutoffs, longer detection times, and limiting HV treatment for PVT and VF not amenable to ATP. As such, programming guidelines recommend faster detection rate cutoffs and longer detection times to reduce avoidable HV therapy.

Also, VTs can be further classified into monomorphic ventricular tachycardia (MVT) and polymorphic ventricular tachycardia (PVT). PVT refers to a malignant ventricular tachyarrhythmia with changing QRS pattern that will either terminate spontaneously (causing syncope if it lasts more than a few seconds) or will deteriorate to ventricular fibrillation (VF), causing cardiac arrest. Defining the etiology of polymorphic VT is of utmost importance because different types of arrhythmias with similar electrocardiographic characteristics respond to different forms of therapy. Furthermore, polymorphic VT often leads to arrhythmic storms, with clusters of VF episodes repeatedly requiring high voltage DC shocks for defibrillation.

MVT refers to a ventricular tachycardia that is defined by the following characteristics: a regular wide QRS complex (e.g., ≥120 milliseconds) tachycardia at a rate greater than 100 beats per minute, and where the consecutive beats have a uniform and stable QRS morphology between successive heart beats. An MVT typically originates from a single focus within a ventricle and is commonly seen in patients with underlying structural heart disease.

Heretofore, methods have been proposed to distinguish between MVT and ventricular fibrillation. For example, an arrhythmia may be classified as MVT or VF based on rate. However, this conventional approach is not able to discriminate between PVT and a slow VF.

It has further been proposed to discriminate high rate polymorphic QRS complexes (both polymorphic VT and polymorphic VF) from high rate monomorphic QRS complexes (both monomorphic VT and monomorphic VF) employing wavelet transform signal processing of the QRS complexes. Wavelet transforms are applied to the sampled amplitude values of a sequence of QRS complexes to develop wavelet transform coefficient (WTC) data sets. At least selected ones of the WTC data sets are processed and comparisons are made to determine a wavelet match score. A determination is made as a function of the wavelet match scores of the series of successive QRS complexes that characterizes the most recent QRS complex as more or less likely to signify polymorphic VT or VF. However, the foregoing wavelet transform based process is highly complex and performs the analysis based on wavelet transformations, not based directly on the measured signals. Also, if the wavelet-based algorithm misses one or more beats during a polymorphic arrhythmia episode, and only evaluates a portion of the beats that may be similar, the potential exists that the wavelet based approach would not be able to differentiate monomorphic arrhythmias from polymorphic arrhythmias.

In another approach, it has been proposed to discriminate between a monomorphic tachyarrhythmia (both monomorphic VT and monomorphic VF) and a polymorphic tachyarrhythmia (both polymorphic VT and polymorphic VF) by examining frequency content and baseline information of an electrocardiogram (EGM) as discriminatory signatures. The frequency content of the QRS complexes is determined using a Slope Distribution Metric (SDM) algorithm and a Slope Distribution Composite (SDC) algorithm. However, the foregoing frequency content-based process is highly complex in order to distinguish both polymorphic VT and VF from monomorphic VT and VF.

A need remains for methods and devices that discriminate between stable (monomorphic) VT and unstable (polymorphic) VT, without the added complexity needed to also distinguish monomorphic VT from polymorphic VT. A simpler discrimination process enables improvements in arrhythmia binning, therapy delivery and ICD outcomes.

US 5,193,535 discloses an implantable cardioverter/defibrillator for discrimination between ventricular tachycardia and ventricular fibrillation. The device is provided with two pairs of electrodes, each pair of electrodes coupled to processing circuitry for identifying a predetermined fiducal point in the electrical signal associated with a ventricular depolarization. The cumulative beat to beat variability of the intervals separating the two identified fiducal points, over a series of detected depolarizations is analyzed. The result of this analysis is used to distinguish between ventricular tachycardia and ventricular fibrillation.

### SUMMARY

The present invention is defined in the independent claims. Further embodiments of the invention are defined in the dependent claims.

In accordance with embodiments herein, an implantable medical device (IMD) is provided. The IMD includes electrodes configured to be located in or about a heart. The electrodes include a bipolar electrode combination that define a bipolar sensing vector. The electrodes include a unipolar electrode combination that define a unipolar sensing vector. The IMD includes sensing circuitry configured to define a first sensing channel coupled to the bipolar electrode combination and a second sensing channel coupled to the unipolar electrode combination. The IMD includes memory to store program instructions and one or more processors configured to implement the program instructions to collect cardiac activity (CA) signals over the first and second sensing channels for a series of beats, identify a sensed interval delta (SID) from the CA signals for at least a portion of the series of beats, the SID representing a timing interval between i) a COI in the CA signals collected over the first sensing channel and ii) the same COI in the CA signals collected over the second sensing channe; and designate the series of beats to correspond to be a polymorphic ventricular tachycardia (PVT) or a monomorphic ventricular tachycardia (MVT) based on the SID.

Optionally, the one or more processors may be further configured to deliver a PVT related therapy or an MVT related therapy based on whether the series of beats are declared to be PVT or MVT. The one or more processors may be further configured to identify an amplitude characteristic of interest (COI) from the CA signals collected over the second sensing channel associated with the bipolar sensing vector for at least a portion of the series of beats, the designation of the series of beats to be PVT or MVT based in part on the COI. The COI may be an R-wave and the SID represents the timing interval between i) a R-wave in the CA signals collected over the first sensing channel and ii) the same R-wave in the CA signals collected over the second sensing channel.

Optionally, the one or more processors may be further configured to compare the SID and the amplitude COI to corresponding SID and COI criteria and based on the comparisons. The one or more processors may designate the current series of beats to be a PVT or MVT. The SID and COI criteria may include SID and COI thresholds, respectively. The one or more processors may be further configured to at least one of a) designate the series of beats to be PVT when the SID and amplitude COI exceed the corresponding SID and COI thresholds, b) designate the series of beats to be MVT when the SID and amplitude COI do not exceed the corresponding SID and COI thresholds, or c) designate a discrimination not possible condition when only one of the SID and amplitude COI exceed the corresponding SID and COI thresholds and another of the SID and amplitude COI do not exceed the corresponding SID and COI thresholds.

Optionally, the first sensing channel may include a first amplifier and a first digital processing circuit. The first amplifier may include input terminals that are coupled to the bipolar electrode combination. The second sensing channel may include a second amplifier and a second digital processing circuit. The second amplifier may include input terminals that are coupled to the unipolar electrode combination.

Optionally, the bipolar and unipolar electrode combinations may include at least one common electrode. The bipolar electrode combination may include first and second electrodes located within or proximate to one or more chambers of the heart. The unipolar electrode combination may include a third electrode located remote from the heart. The one or more processors may be configured to store the designation of the PVT or MVT. The IMD may further comprise a telemetry circuitry configured to downloaded the designation of the PVT or MVT, from the IMD, to an external device.

In accordance with embodiments herein, a method is provided. The method identifies a sensed interval delta (SID) from cardiac activity (CA) signals collected over a first sensing channel coupled to a bipolar electrode combination and a second sensing channel coupled to a unipolar electrode combination for at least a portion of a series of beats. The SID represents a timing interval between i) a COI in the CA signals collected over the first sensing channel and ii) the same COI in the CA signals collected over the second sensing channel. The method also comprisesdesignating the series of beats to correspond to be a polymorphic ventricular tachycardia (PVT) or a monomorphic ventricular tachycardia (MVT) based on the SID.

Optionally, the method may comprise identifying an amplitude characteristic of interest (COI) from the CA signals collected over the second sensing channel associated with the bipolar sensing vector for at least a portion of the series of beats, the designating of the series of beats to be PVT or MVT based in part on the COI.

The COI may be an R-wave and the SID represents the timing interval between i) a R-wave in the CA signals collected over the first sensing channel and ii) the same R-wave in the CA signals collected over the second sensing channel.

Optionally, the method may compare the SID and the amplitude COI to corresponding SID and COI criteria and based on the comparisons, designating the current series of beats to be a PVT or MVT. The SID and COI criteria may include SID and COI thresholds, respectively. The method may further comprise designating the series of beats to be PVT when the SID and amplitude COI exceed the corresponding SID and COI thresholds. The SID and COI criteria may include SID and COI thresholds, respectively. The method may further comprise designating the series of beats to be MVT when the SID and amplitude COI do not exceed the corresponding SID and COI thresholds.

Optionally, the SID and COI criteria may include SID and COI thresholds, respectively. The method may further comprise designating a discrimination not possible condition when only one of the SID and amplitude COI exceed the corresponding SID and COI thresholds and another of the SID and amplitude COI do not exceed the corresponding SID and COI thresholds. The first and second sensing channels may utilize at least one common electrode.

This disclosure proposes an IMD and method to track and evaluate the sensed events during a ventricular arrhythmia and stratify them between polymorphic or monomorphic event. The invention takes advantage of peak R-wave amplitudes and sensed interval delta in the device bipolar and unipolar signals, collected throughout the arrhythmia episode, from which the IMD or method can determine the therapy to deliver for the identified arrhythmia.

### BRIEF DESCRIPTION OF THE DRAWINGS

Figure 1 illustrates an IMD and external device coupled to a heart in a patient and implemented in accordance with one embodiment.
Figure 2 illustrates a simple block diagram of at least a portion of the circuitry within the IMD.
Figure 3 illustrates examples of CA signals collected along primary and second sensing channels for a PVT and MVT.
Figure 4A illustrates a process for discriminating between PVT and MVT episodes based on one or more COI from a series of beats, in accordance with embodiments herein.
Figure 4B illustrates a process for discriminating between PVT and MVT episodes based the SID from a series of beats, in accordance with embodiments herein.
Figure 5 illustrates an example of CA signals and that are collected over a bipolar first sensing channel and a unipolar second sensing channel during a PVT and an MVT.
Figure 6 illustrates an example of CA signals 602 and 604 that are collected over a bipolar first sensing channel and a unipolar second sensing channel.
Figure 7 illustrates a block diagram of the IMD 100 formed in accordance with embodiments herein.

### DETAILED DESCRIPTION

It will be readily understood that the components of the embodiments as generally described and illustrated in the figures herein, may be arranged and designed in a wide variety of different configurations in addition to the described example embodiments. Thus, the following more detailed description of the example embodiments, as represented in the figures, is not intended to limit the scope of the embodiments, as claimed, but is merely representative of example embodiments.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, etc. In other instances, well-known structures, materials, or operations are not shown or described in detail to avoid obfuscation. The following description is intended only by way of example, and simply illustrates certain example embodiments.

The methods described herein may employ structures or aspects of various embodiments (e.g., IMDs, systems and/or methods) discussed herein. In various embodiments, certain operations may be omitted or added, certain operations may be combined, certain operations may be performed simultaneously, certain operations may be performed concurrently, certain operations may be split into multiple operations, certain operations may be performed in a different order, or certain operations or series of operations may be re-performed in an iterative fashion. It should be noted that, other methods may be used, in accordance with an embodiment herein. Further, wherein indicated, the methods may be fully or partially implemented by one or more processors of one or more devices or systems. While the operations of some methods may be described as performed by the processor(s) of one device, additionally, some or all of such operations may be performed by the processor(s) of another device described herein.

The term "Ventricular tachycardia" shall mean a ventricular arrhythmia that continues to make the heartbeat regularly but goes so fast that the heart never gets a chance to fill with blood. There's not an opportunity to build up the pressure, so the blood stops flowing.

The term "Ventricular fibrillation" is a form of heart rhythm disturbance (dysrhythmia) that causes cardiac arrest, wherein the heart stops beating normally and simply begins quivering uncontrollably.

The terms "characteristic of interest" and "COI" shall mean a characteristic of interest in a cardiac cycle or heartbeat that is manifest or otherwise apparent in the CA signals collected for the cardiac cycle or heartbeat. For example, a COI may be a peak of a ventricular contract, also referred to as a peak of an R-wave.

The phrase "same COI" shall mean a characteristic of interest in a single/common cardiac cycle that is manifest in CA signals measured over multiple sensing channels. The same COI may manifest itself or otherwise appear differently in first and second CA signals collected over first and second sensing channels. For example, when a COI in the CA signals collected over the first sensing channel corresponds to a peak of a ventricular contraction in a specific cardiac cycle, the COI may appear at a first point in time as a peak in the CA signals with certain amplitude. The CA signals leading upto and following the peak may have first increasing and decreasing slopes. The phrase "the same COI" shall mean a peak of the same ventricular contract in the same cardiac cycle, but which will manifest itself in a different manner in the CA signals collected over a different sensing channel (e.g., have a different peak amplitude, occur at a different point in time, have a different increasing and/or decreasing slope).

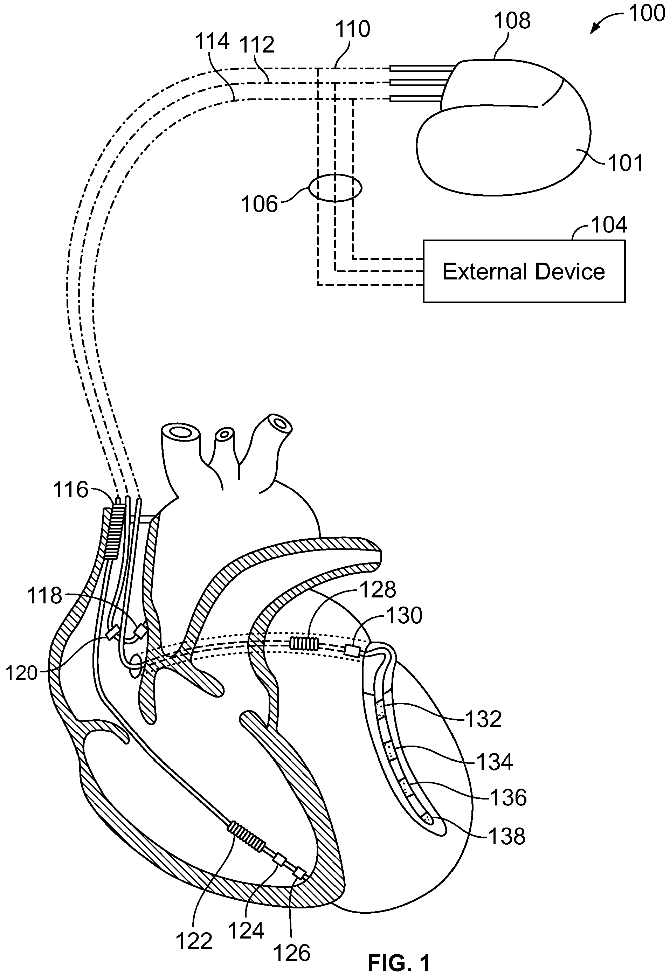

Figure 1 illustrates an IMD 100 and external device 104 coupled to a heart in a patient and implemented in accordance with one embodiment. The external device 104 may be a programmer, an external defibrillator, a workstation, a portable computer, a personal digital assistant, a cell phone, a bedside monitor and the like. The IMD may represent a cardiac monitoring device, pacemaker, cardioverter, cardiac rhythm management device, defibrillator and the like, implemented in accordance with one embodiment of the present invention. The IMD 100 may be a dual-chamber stimulation device capable of treating both fast and slow arrhythmias with stimulation therapy, including cardioversion, defibrillation, anti-tachycardia pacing and pacing stimulation, as well as capable of detecting heart failure (HF), evaluating HF severity, tracking the progression of HF, and controlling the delivery of therapy and warnings in response thereto. The IMD 100 may be controlled to sense atrial and ventricular waveforms of interest, discriminate between two or more ventricular waveforms of interest, deliver stimulus pulses or shocks, and inhibit application of a stimulation pulse to a heart based on the discrimination between the waveforms of interest and the like. Exemplary structures for the IMD 100 are discussed and illustrated in the drawings herewith.

The IMD 100 includes a housing 101 that is joined to a header assembly 109 that holds receptacle connectors connected to a right ventricular lead 110, a right atrial lead 112, and a coronary sinus lead 114, respectively. The leads 112, 114 and 110 measure cardiac signals of the heart. The right atrial lead 112 includes an atrial tip electrode 118 and an atrial ring electrode 120. The coronary sinus lead 114 includes a left atrial ring electrode 128, a left atrial coil electrode 130 and one or more left ventricular electrodes 132-138 (e.g., also referred to as P1, M1, M2 and D1) to form a multi-pole LV electrode combination. The right ventricular lead 110 includes an RV tip electrode 126, an RV ring electrode 124, an RV coil electrode 122, and an SVC coil electrode 116. The leads 112, 114 and 110 detect IEGM signals that are processed and analyzed as described herein. The leads 112, 114 and 110 also delivery therapies as described herein.

As explained herein, the electrodes are configured to be located in or about a heart. The electrodes include a bipolar electrode combination that defines a bipolar sensing vector. The electrodes further include a unipolar electrode combination that defines a unipolar sensing vector. For example, the bipolar electrode combination may include the RV tip electrode 126 and RV ring electrode 124, while the unipolar electrode combination may include the RV tip electrode 126 and the housing 101 as a CAN electrode. Optionally, the bipolar electrode combination may include alternatively combinations the RV tip, coil and ring electrodes 126, 122, and 124. Optionally, the bipolar electrode combination may include alternatively combinations of RV and LV electrodes such as one or more of the RV tip electrode 126 and/or RV ring electrode 124 and one or more of the LV electrode 132-138. Optionally, the unipolar electrode combination may include the CAN electrode and one of the RV tip, coil or ring electrodes 126, 122 and 124. Optionally, the unipolar electrode combination may include the CAN electrode and one of the LV electrodes 132-138.

The unipolar and bipolar sensing channels collect electrical potential changes indicative of electrical activity in partially overlapping regions of the heart. For example, the bipolar sensing channel may collect electrical potential changes indicative of electrical activity substantially entirely in a right ventricle (RV), while the unipolar sensing channel collects electrical potential changes indicative of electrical activity partially in the RV but partially in one or more other chambers of the heart (e.g., left ventricle, left atrium, right atrium).

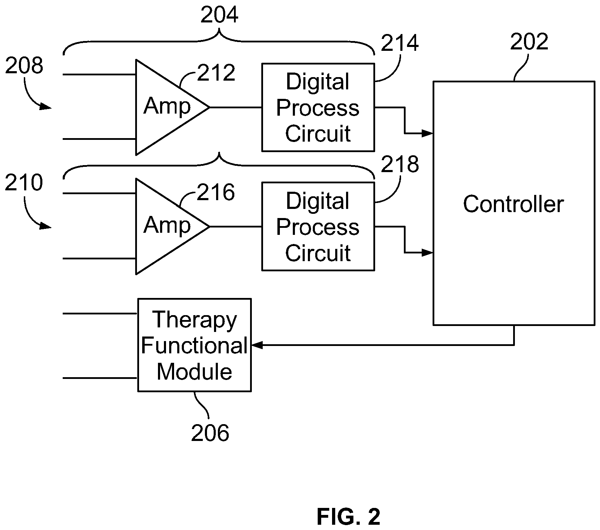

Figure 2 illustrates a simple block diagram of at least a portion of the circuitry within the IMD 100. The IMD 100 includes a controller 202 that may be coupled to sensing circuitry 204 and a therapy functional module 206. The controller 202 includes one or more processors that, when executing program instructions, are configured to perform operations described herein. The one or more processors also utilize or communicate with various other electronic components, firmware, software, and the like that generally perform sensing and pacing functions (as generally denoted by a therapy functional block 206). While the examples herein are provided for pacing, ATP and defibrillation functions, the IMD could be programmed to perform cardiac rhythm therapy, and the like. The sensing circuitry 204 is configured to define a first sensing channel 208 coupled to a bipolar electrode combination and to define a second sensing channel 210 coupled to a unipolar electrode combination.

The first sensing channel 208 includes an amplifier 212 and a digital processing circuit 214. The amplifier 212 includes input terminals that are coupled to a bipolar electrode combination. For example, the bipolar electrode combination may include a tip electrode and a coil electrode. For example, the tip electrode may be electrode 126 in Figure 1, located proximate to the apex of the right ventricle (RV), while the coil electrode may be coil electrode 122 in Figure 1 located at an intermediate point within the RV. The amplifier 212 outputs an amplified analog difference between the electrical potentials at the input terminals (e.g., the difference between the electrical potentials at the tip and coil electrodes 126 and 122). The analog difference is supplied to the digital processing circuit 214 which digitizes and otherwise processes (e.g., filters) the incoming analogue signal to form a digital processed signal.

The second sensing channel 216 includes an amplifier 218 and a digital processing circuit 220. The amplifier 218 includes input terminals that are coupled to a unipolar electrode combination. For example, the unipolar electrode combination may include the RV coil electrode and a CAN electrode. For example, the RV coil electrode may be electrode 122 in Figure 1, located at an intermediate point within the RV, while the CAN electrode is formed by the housing 101 of the IMD 100. The amplifier 216 outputs an amplified analog difference between the electrical potentials at the input terminals (e.g., the difference between the electrical potentials at the RV coil and CAN electrodes 122 and 101). The analog difference is supplied to the digital processing circuit 218 which digitizes and otherwise processes (e.g., filters) the incoming analogue signal to form a digital processed signal.

The amplifiers 212 and 216 output an electrical signal indicative of changes in an electrical potential along corresponding sensing vectors between two or more corresponding electrodes. The CA signals are not impedance measurements. In other words, the CA signals are non-impedance electrical CA signals.

While the example of Figure 2 illustrated as circuitry within a transvenous IMD, it is understood that the circuitry may be implemented by a leadless IMD, a subcutaneous IMD and the like. For example, the amplifier 212 may have input terminals connected to tip and ring electrodes located proximate to a distal end and/or intermediate segment of a subcutaneous lead connected to a subcutaneous IMD in a bipolar sensing configuration, while the amplifier 216 may have input terminals connected to the same or different tip or ring electrodes and a housing of the subcutaneous IMD. Additionally, or alternatively, the amplifier 212 and digital processing circuit 214 may be implemented in a leadless IMD (to provide a bipolar sensing channel), while the amplifier 216 and digital processing circuit 218 may be implemented in a subcutaneous IMD (to provide a unipolar sensing channel).

### Discrimination Between Monomorphic VT and Polymorphic VT

In accordance with new and unique aspects herein, simple methods and systems are described to track and evaluate sensed events during a ventricular arrhythmia and classify the events as polymorphic VT (PVT) or monomorphic VT (MVT).

In general, the unipolar electrode combination has one pole (cathode or negative stimulating pole) in contact with cardiac tissue, and the other pole (anode or positive pole) outside of the heart, either in subcutaneous tissue or on the surface of the body. The bipolar electrode combination has both the cathode (sometimes called its distal or tip pole) and the anode (proximal or ring pole) at the cardiac tissue being stimulated. Bipolar and unipolar electrodes are not equivalent in transmitting the cardiac electrograms. Only the electrical events at the tip pole describe the unipolar electrogram, with the remote anode contributing negligible voltage as its location is extracardiac. The bipolar electrode combination exhibits a large anodal voltage (ring signal), similar in magnitude to the tip signal, but the resulting electrogram is also dependent upon the orientation of the electrode within the heart. Embodiments herein take advantage of the differences in the electrogram signals sensed at unipolar and bipolar electrode combinations.

The methods and systems take advantage of i) variability criterion and/or sensed interval delta (SID) criterion that appear different within CA signals collected along different sensing vectors and utilizing different sensing polarities. The variability criterion represent variability within peak R-wave amplitudes. The SID criterion represent a sensed interval delta between a characteristic of interest in cardiac activity signals collected along primary (e.g., bipolar) and secondary (e.g., unipolar) sensing channels. Based on either or both of the variability and/or SID criteria, the methods and systems classify VT events as polymorphic or monomorphic and determine a corresponding action or therapy to deliver for the identified type of VT.

Based on the classification of MVT or PVT, the system and method may take different actions depending upon the type of IMD. For example, the IMD may represent a transvenous or subcutaneous IMD that is configured to treat MVT and PVT. The treatment may include one or more HV shocks, one or more MV shocks, ATP therapy and the like. Additionally or alternatively, the IMD may withhold treatment for a period of time and allow a MVT to self-terminate. If the MVT does not self-terminate, the IMD may then deliver one or more types of therapy. For example, a PVT may warrant a HV shock, while a MVT may be treated with ATP, a MV shock and/or afforded a period of time to self-terminate.

The IMD collects CA signals along primary and secondary sensing channels that utilize different sensing vectors and electrode polarity configurations.

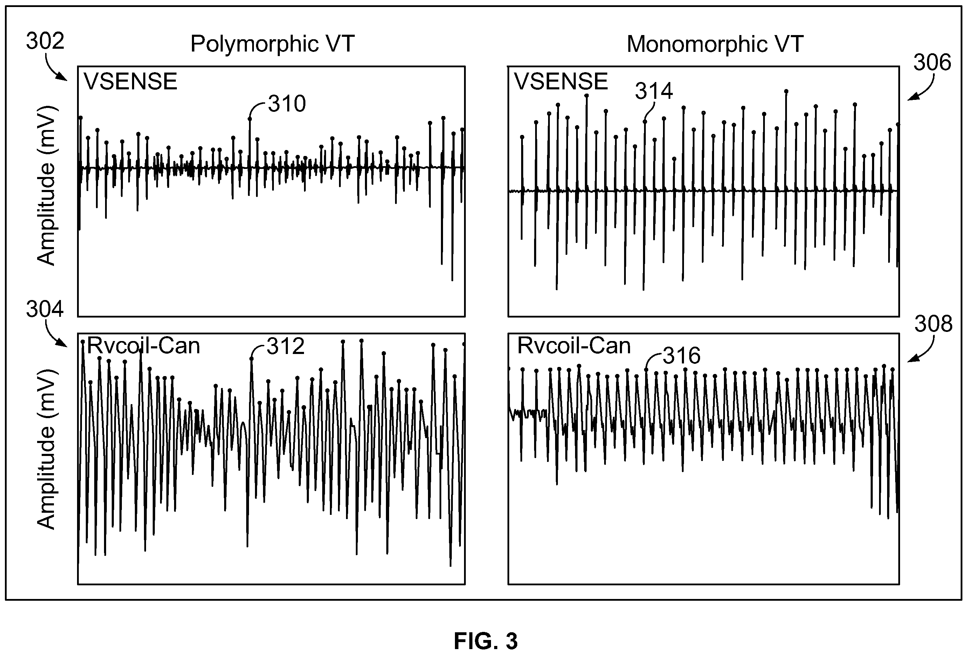

Figure 3 illustrates examples of CA signals (e.g., EGMs) collected along primary and second sensing channels for a PVT and MVT. In accordance with embodiments herein, methods and systems discriminate between an unstable\polymorphic and a stable\monomorphic VT done using one or both of an amplitude COI and/or an SID. In Figure 3, panel 302 illustrates CA signals for a series of beats where the CA signals are collected along a sensing vector that utilizes a bipolar electrode configuration (e.g., two RV electrodes, one RV and one LV electrode, one RV electrode and two or more LV electrodes, two or more RV electrodes and one LV electrode, one or more RA electrodes and one or more RV electrodes, etc.). The CA signals correspond to a PVT episode, in which each beat has a peak R-wave amplitude as denoted by the dots 310. Panel 304 illustrates CA signals for the same series of beats as in panel 302, but with the CA signals collected along a sensing vector that utilizes a unipolar electrode configuration (e.g., one or more RV electrodes and the CAN electrode, one or more LV electrodes and the CAN electrode, etc.). The CA signals include beats with R-wave peak amplitudes as denoted by the dots 312. By comparing the CA signals in 302 and 304, it can be seen that the PVT CA signals collected along the unpolar sensing channel exhibit greater peak to peak amplitudes as compared to the CA signals collected along the bipolar sensing channel.

Panel 306 illustrates CA signals for a series of beats where the CA signals are collected along a sensing vector that utilizes a bipolar electrode configuration. The CA signals correspond to an MVT episode, in which each beat has a peak R-wave amplitude as denoted by the dots 314. Panel 308 illustrates CA signals for the same series of beats as in panel 306, but with the CA signals collected along a sensing vector that utilizes a unipolar electrode configuration. The CA signals include beats with R-wave peak amplitudes as denoted by the dots 316. By comparing the CA signals in panels 302 and 304, it can be seen that the MVT CA signals collected along the bipolar sensing channel exhibit greater peak to peak amplitudes as compared to the CA signals collected along the unipolar sensing channel.

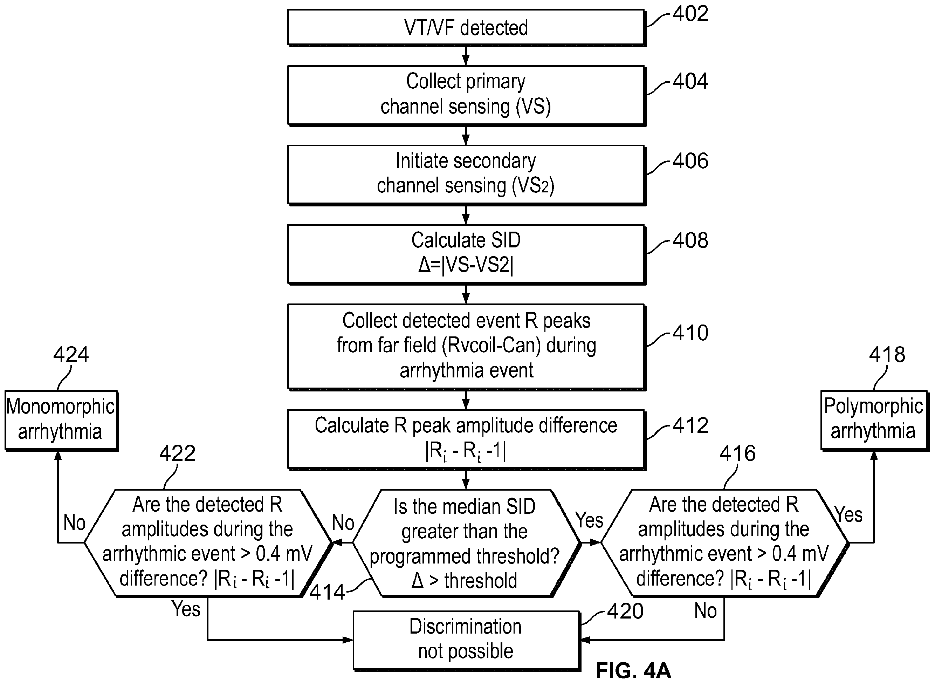

Figure 4A illustrates a process for discriminating between PVT and MVT episodes based on one or more COI from a series of beats, in accordance with embodiments herein. The operations of Figure 4A may be implemented in whole or in part by one or more processors of an IMD, an external device, a bedside monitor, a remote server or other computing device. In a particular embodiment, the operations of Figure 4A are implemented in whole in an external device, a bedside monitor, a remote server or other computing device. Hence, in such a particular embodiment, the operations are not conducted by any device implanted in a subject.

At 402, the one or more processors analyze the CA signals collected over one or more sensing channels. For example, the CA signals may be collected over a bipolar sensing channel, a unipolar sensing channel or one or more other sensing channels. The one or more processors analyze the CA signals to determine whether VT or VF are indicated. Various arrhythmia discrimination algorithms may be used to analyze the CA signals. Additionally or alternatively, the analysis at 402 may be based on another type of signal other than a CA signal. For example, the VT/VF may be detected based on heart sounds, impedance signals and the like.

AT 404 and 406, the one or more processors manage obtaining CA signals over the first and second sensing channels for a series of beats. The series of beats corresponds to the series of beats for which the VT/VF was determined at 402. For example, at 404 and 406, the one or more processors may merely access segments of CA signals stored in memory in connection with the first and second sensing channels. All CA signals collected over the first sensing channel may be stored in a first first-in-first-out (FIFO) buffer and all CA signals collected over the second sensing channel may be stored in a second FIFO buffer. When the buffers are full, the oldest/first data written to the buffer is written over by the newest data. The obtaining operations at 404 and 406 may involve moving segments of CA signals from the first and second buffers to longer-term memory for storage and analysis as described herein.

Optionally, the obtaining operations at 404 and 406 may include sensing/collection of new CA signals that are separate and occur after the CA signals analyzed at 402. For example, it may be desirable to perform the analysis at 404 to 424 based on CA signals that are separate and occur after the CA signals utilized to initially declare the VT/VF. As a further example, the VT/VF episode may be initially determined based on a type of biological data other than a CA signal (e.g., heart sounds, impedance signals). Accordingly, the CA signals analyzed at 404-424 represent a confirmation of a separately declared VT/VF, as well as a discriminator between PVT and MVT.

Optionally, the obtaining operations at 404 and 406 may be performed in parallel with the VT/VF determining operation at 402. For example, new CA signals may be sensed at 404 and 406, and analyzed at 402 to determine whether a VT/VF episode is present.

At 408, the one or more processors identify a sensed interval delta (SID) from the CA signals for at least a portion of the series of beats. The one or more processors identify a COI in the CA signals collected over the first sensing channel and identify the same COI in the CA signals collected over the second sensing channel. The one or more processors calculate a time interval, as the SID, between i) the COI in the CA signals collected over the first sensing channel and ii) the same COI in the CA signals collected over the second sensing channel.

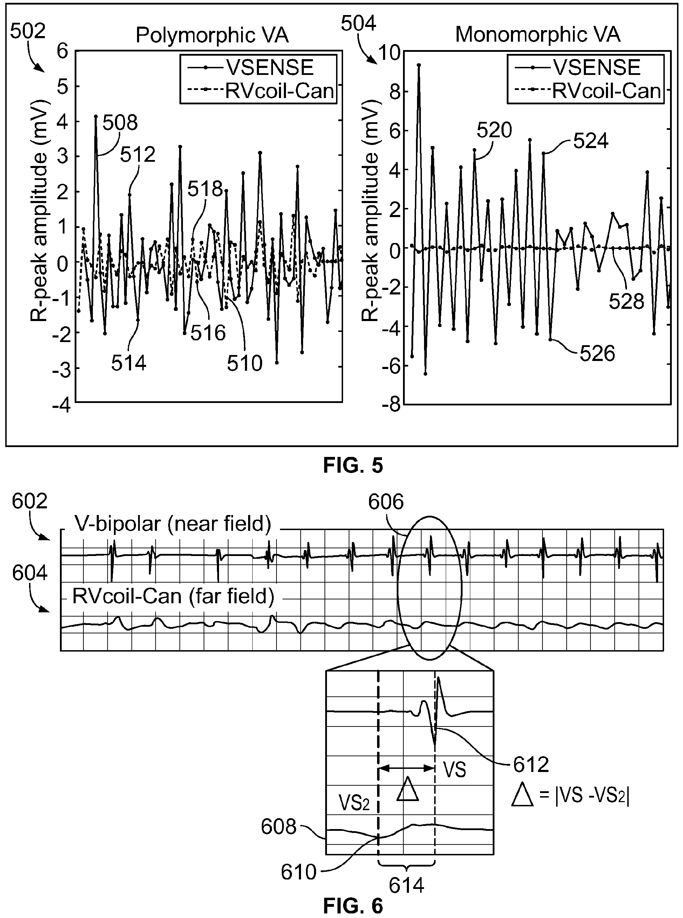

The identification at 408 is described further in connection with Figure 6. Figure 6 illustrates an example of CA signals 602 and 604 that are collected over a bipolar first sensing channel and a unipolar second sensing channel. The first sensing channel is also referred to as a near field sensing channel as the bipolar sensing channel is more sensitive (relative to the unipolar sensing channel) to activity in the proximal area surrounding the bipolar electrode configuration. The second sensing channel is also referred to as a far field sensing channel as the unipolar sensing channel is more sensitive (relative to the bipolar sensing channel) to activity in the area remote from the unipolar electrode configuration.

At 408, the one or more processors analyze the CA signals 602, 604 in connection with each beat or cardiac cycle. The beat 606 is enlarged in panel 608 for further explanation. In panel 608, the one or more processors identify a peak deflection 612 in the CA signals 602 (for the bipolar electrode configuration). The one or more processors identify a peak deflection 610 in the CA signals 604 (for the unipolar electrode configuration). The peak deflections 610, 612 represent the "same COI" as both deflections represent a manifestation of a single event in a single cardiac cycle. The peak deflection 610, 612 manifests itself or otherwise appears differently in first and second CA signals 602, 604 collected over first and second sensing channels. For example, peak deflection 612 in the CA signals 602 exhibits a much greater peak to peak deflection as compared to other deflections (e.g., associated with the P-wave or T-wave) in the CA signal 602 and as compared to the peak defection 610 in the CA signals 604. In addition, the peak deflection 612 exhibits much greater slope, with a very short transition time between peaks, (as compared to the CA signals 604) when transitioning from the isoelectric potential to the peak negative value, then to the peak positive value, then returning to the isoelectric potential.

The peak deflection 610 in the CA signals 604 exhibits a much small peak to peak deflection (as compared to the peak deflection 612). In addition, the peak deflection 610 exhibits a much lower (more gradual) slope, with a relatively long transition time between peaks, (as compared to the CA signals 602) when transitioning from the isoelectric potential to the peak negative value, then to the peak positive value, then returning to the isoelectric potential. The one or more processors record the points in time VS2 and VS when the peaks deflections 610 and 612, respectively, occur (as noted by the vertical dashed lines). The one or more processors then calculate the time interval or SID 614 between the peak deflections (Δ = Absolute value(VS-VS2)).

In the foregoing example, the COI represents a peak ventricular contraction that manifests itself as peak deflections 610, 612. Additionally, or alternatively, the COI may represent another feature of the cardiac cycle, or a combination of features in the cardiac cycle. For example, the COI may correspond to a point where the CA signals 602, 604 have a maximum positive of negative slope (e.g., maximum derivative). As another example, the COI may correspond to a center of a QRS complex in the CA signals 602, 604. As another example, the COI may correspond to a start of a QRS complex in the CA signals 602, 604.

Returning to Figure 4A, at 410, the one or more processors collect the event R-wave peaks for at least a portion of the cardiac cycle, for the CA signals from the far field, unipolar sensing channel. The CA signals utilized at 410 may be the same CA signals utilized at 402-408, or may be a new set of CA signals, but collected while the patient is still exhibiting the arrhythmia episode.

The operation at 410 is described in more detail in connection with Figure 5. Figure 5 illustrates an example of CA signals that are collected over a bipolar first sensing channel and a unipolar second sensing channel during a PVT and an MVT. Panel 502 corresponds to a PVT episode, while panel 504 corresponds to an MVT episode. In panel 502, CA signals 508 and 510 are collected over a bipolar first sensing channel and a unipolar second sensing channel, respectively. The CA signals 508 (over the bipolar sensing channel), during a PVT, exhibit a somewhat greater peak to peak amplitude difference (amplitude COI) (e.g., between successive peaks 512 and 514), as compared to the peak to peak (P-P) amplitude difference (e.g., between successive peaks 516 and 518) in the PVT CA signals 510 (over the unipolar sensing channel).

However, the P-P amplitude difference is much more notable, during an MVT. During the MVT, the CA signals 520 (over the bipolar sensing channel), exhibit a much greater peak to peak amplitude difference (e.g., between peaks 524 and 526), as compared to the P-P amplitude difference in the MVT CA signals 528 (over the unipolar sensing channel). In fact, the MVT CA signals 528 exhibit very little P-P amplitude difference between successive beats. Accordingly, the P-P amplitude difference in the CA signals sensed over the unipolar sensing channel yield a good indicator for MVT v. PVT. For example, the CA signals 510, during a PVT, for the unipolar sensing channel exhibits a normalized P-P amplitude difference of 2 (+1 to -1), while the CA signals 528, during a MVT, for the unipolar sensing channel exhibits a normalized P-P amplitude difference of less than 0.5. More generally, it has been found that the P-P amplitude difference during an MVT is between 0 and 0.2, as measured over the unipolar sensing channel, while the P-P amplitude difference during a PVT is between 0.2 and 0.6, as measured over the bipolar sensing.

Returning to Figure 4A, at 412, the one or more processors calculate a relative feature of interest between the R-wave peaks. For example, the one or more processors may calculate a maximum P-P amplitude difference in an amplitude of R-wave peaks between successive beats (e.g., Rᵢ-Rᵢ₋₁), where Rᵢ represents an amplitude of an R-wave peak for a current beat and Rᵢ₋₁ represents an amplitude of an R-wave peak for an immediately preceding beat.

At 414 to 424, the one or more processors compare the SID and the amplitude COI to corresponding criteria and based on the comparisons, the one or more processors designate the current series of beats to be a PVT or MVT. The designation may be stored and later downloaded from the IMD to an external device. Additionally, or alternatively, the designation of a PVT or MVT may be used to determine a therapy. The therapy may be delivered by the same IMD that collects and analyzed the CA signals and/or the therapy may be delivered by another IMD. For example, a transvenous IMD may collect the CA signals, and then deliver an appropriate therapy. Additionally, or alternatively, a subcutaneous IMD and/or leadless IMD may collect and analyze the CA signals, and then deliver the therapy. Additionally, or alternatively, a subcutaneous, leadless or transvenous IMD, or implantable cardiac monitor, may collect the CA signals over a bipolar first sensing channel, while a different subcutaneous, leadless or transvenous IMD, or implantable cardiac monitor collects the CA signals over a unipolar second sensing channel. One or more of the subcutaneous, leadless or transvenous IMD, or ICM may analyze the CA signals and designate the series of beats to be a PVT or MVT. One or more of the subcutaneous, leadless or transvenous IMD may deliver a PVT or MVT related therapy.

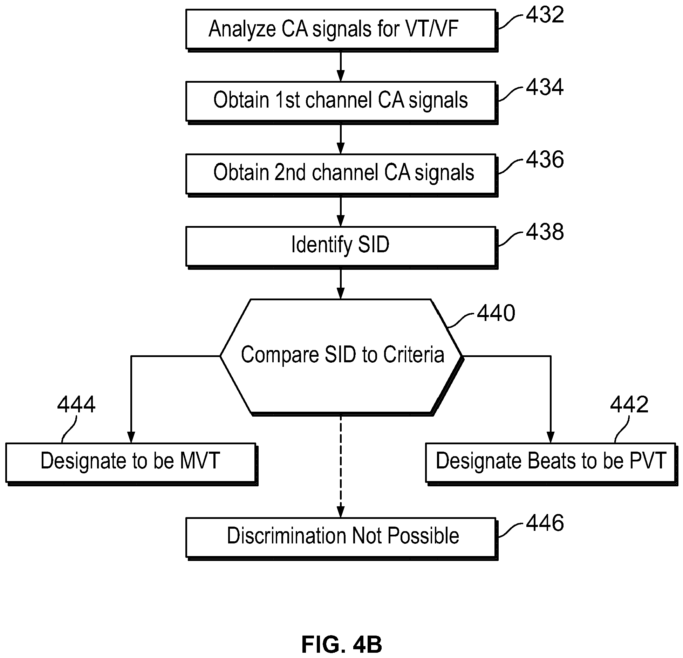

More specifically, in the embodiment of Figure 4, at 414, the one or more processors compare the SID to an SID criterion. For example, the process determines whether a median SID is greater than a programmed SID threshold (e.g., Δ>SID threshold). The SID criterion may be programmed in various manners. For example, the SID may be between 10ms and 50ms in length, and more preferably between 15ms and 40ms. An SID threshold may be programmed to be 15ms or greater. At 414, when the SID (e.g., median SID) is greater than the SID threshold, flow moves to 416. When the SID is less than or equal to the SID threshold, flow moves to 422.

At 416, the one or more processors compare the amplitude COI to an amplitude COI criterion. For example, the amplitude COI may represent a maximum difference in an amplitude of R-wave peaks between successive beats (e.g., Rᵢ-Rᵢ₋₁), where Rᵢ represents an amplitude of an R-wave peak for a current beat and Rᵢ₋₁ represents an amplitude of an R-wave peak for an immediately preceding beat. Additionally, or alternatively, the amplitude COI may represent a maximum difference in a median amplitude between first and second sets of beats (e.g., first set of 3-7 beats and second set of 3-7 beats). The amplitude COI criterion may be programmed in various manners. For example, the amplitude COI may be an amplitude difference of between 0.1mV and 1.2mV between peak amplitudes of successive R-wave peaks. More preferably, the amplitude difference may be between 0.2mV and 0.6mV between peak amplitudes of successive R-wave peaks. At 416, when the amplitude COI (e.g., difference) is greater than the COI threshold, flow moves to 418. When the amplitude COI is equal to or less than the COI threshold, flow moves to 420.

At 418, the SID and amplitude COI have both satisfied the corresponding SID and COI criteria (e.g., thresholds) and accordingly, the one or more processors designate the series of beats to correspond to be a polymorphic ventricular tachycardia.

At 420, the SID criterion is satisfied, but the COI criterion is not satisfied. Accordingly, the one or more processors determine that the process cannot discriminate between PVT or MVT for the series of beats. Accordingly, the series of beats is left unclassified or designated as a "discrimination not possible condition."

Returning to 414, when the SID criterion is not satisfied, flow moves to 422. At 422, the one or more processors apply the amplitude COI criterion to determine whether the amplitude COI is greater than the COI threshold. The test at 422 may be the same as the test at 416. Alternatively, the COI threshold at 422 may differ from the COI threshold applied at 416. For example, at 416, a COI threshold of 0.2mV may be programmed, whereas at 422 the COI threshold may be programmed to a higher lor lower amplitude difference (e.g., 0.3mV or 0.1mV). it may be desirable to use different thresholds at 416 and 422 given that, at 416, the SID criterion is satisfied, but at 422, the SID criterion is not satisfied.

At 422, when the amplitude COI is greater than the COI threshold, flow moves to 420. When the amplitude COI is equal to or less than the COI threshold, flow moves to 424. At 424, the SID criterion is not satisfied, and the amplitude COI criterion is not satisfied. Accordingly, the one or more processors designate the series of beats to correspond to be a monomorphic ventricular tachycardia. Alternatively, when flow moves from 422 to 420, the SID criterion is not satisfied, and the amplitude COI criterion is satisfied. Accordingly, the series of beats is left unclassified or designated as "discrimination not possible condition."

Figure 4B illustrates a process for discriminating between PVT and MVT episodes based the SID from a series of beats, in accordance with embodiments herein. The operations of Figure 4B may be implemented in whole or in part by one or more processors of an IMD, an external device, a bedside monitor, a remote server or other computing device.

At 432, the one or more processors analyze the CA signals collected over one or more sensing channels. The CA signals are collected over a bipolar sensing channel and over a unipolar sensing channel. The one or more processors analyze the CA signals to determine whether VT or VF are indicated. At 434 and 436, the one or more processors manage obtaining CA signals over the first and second sensing channels for a series of beats. The series of beats corresponds to the series of beats for which the VT/VF was determined at 432. Optionally, the obtaining operations at 434 and 436 may include sensing/collection of new CA signals that are separate and occur after the CA signals analyzed at 432. Optionally, the obtaining operations at 434 and 436 may be performed in parallel with the VT/VF determining operation at 432.

At 438, the one or more processors identify a sensed interval delta (SID) from the CA signals for at least a portion of the series of beats. The one or more processors identify a COI in the CA signals collected over the first sensing channel and identify the same COI in the CA signals collected over the second sensing channel. The one or more processors calculate a time interval, as the SID, between i) the COI in the CA signals collected over the first sensing channel and ii) the same COI in the CA signals collected over the second sensing channel.

At 440, the one or more processors compare the SID to an SID criterion. For example, the process determines whether a median SID is greater than a programmed SID threshold (e.g., Δ>SID threshold). The SID criterion may be programmed in various manners. For example, the SID may be between 10ms and 50ms in length, and more preferably between 15ms and 40ms. An SID threshold may be programmed to be 15ms or greater. When the SID is greater than the SID threshold, flow moves to 442. When the SID is less than or equal to the SID threshold, flow moves to 444.

At 442, the one or more processors designate the series of beats to correspond to be a polymorphic ventricular tachycardia.

Alternatively, when flow moves to 444, the one or more processors designate the series of beats to correspond to be a monomorphic ventricular tachycardia.

Optionally, more than one threshold may be used at 440. For example, when the SID exceeds a first threshold, flow may move to 442, where the series of beats are designated PVT. When the SID is below a second threshold, flow may move to 444, where the series of beats are designated MVT. When the SID is between the first and second thresholds, flow may move to 446. At 446, the one or more processors may leave the series of beats unclassified or designated as "discrimination not possible condition."

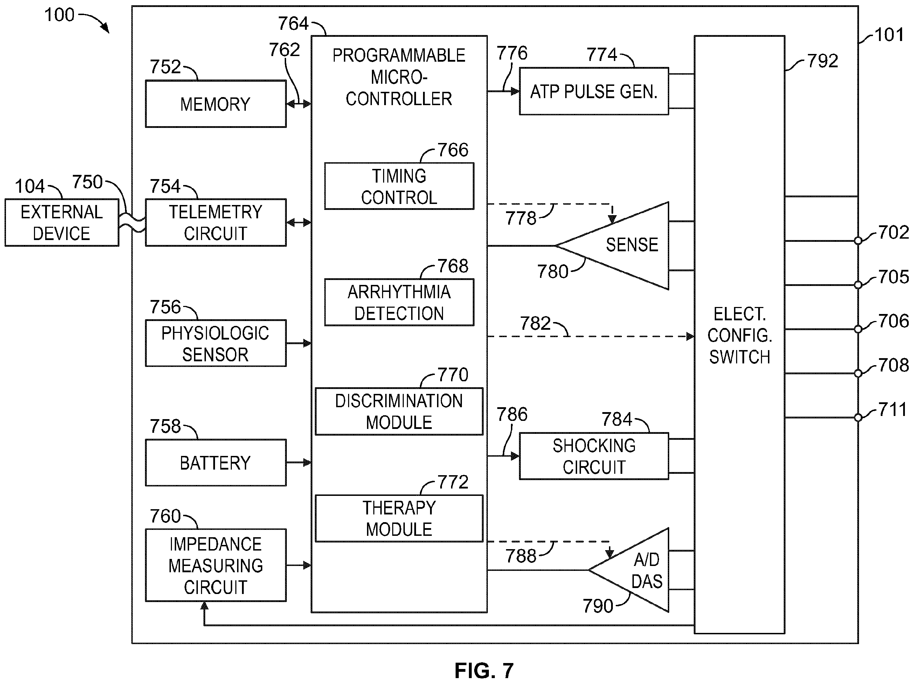

Figure 7 illustrates a block diagram of the IMD 100 formed in accordance with embodiments herein. The housing 101 (which is often referred to as the "can", "case", "encasing", or "case electrode") may be programmably selected to act as the return electrode for certain stimulus modes. Housing 101 further includes a connector (not shown) with a plurality of terminals 702, 705, 706, 708, and 711. The terminals may be connected to electrodes that are located in various locations within and about the heart. For example, the terminals may include: a terminal 702 to be coupled to an first electrode (e.g., a tip electrode) located in a first chamber; a terminal 705 to be coupled to a second electrode (e.g., tip electrode) located in a second chamber; a terminal 706 to be coupled to an electrode (e.g., ring) located in the first chamber; a terminal 708 to be coupled to an electrode located (e.g., ring electrode) in the second chamber; and a terminal 711 to be coupled to an electrode (e.g., coil) located in the SVC. The type and location of each electrode may vary. For example, the electrodes may include various combinations of ring, tip, coil and shocking electrodes and the like. The electrodes include a bipolar electrode combination that defines a bipolar sensing vector and a unipolar electrode combination that defines unipolar sensing vector. The bipolar and unipolar electrode combinations may include at least one common electrode. For example, the bipolar electrode combination may include first and second electrodes located within or proximate to one or more chambers of the heart, and the unipolar electrode combination may include a third electrode located remote from the heart.

The IMD 100 includes a programmable microcontroller 764 that controls various operations of the IMD 100, including cardiac monitoring and stimulation therapy. Microcontroller 764 includes a microprocessor (or equivalent control circuitry), RAM and/or ROM memory, logic and timing circuitry, state machine circuitry, and I/O circuitry.

IMD 100 further includes a first chamber pulse generator 774 that generates stimulation pulses for delivery by one or more electrodes coupled thereto. The pulse generator 774 is controlled by the microcontroller 764 via control signal 776. The pulse generator 774 is coupled to the select electrode(s) via an electrode configuration switch 792, which includes multiple switches for connecting the desired electrodes to the appropriate I/O circuits, thereby facilitating electrode programmability. The switch 792 is controlled by a control signal 786 from the microcontroller 764. In the example of Figure 7, a single pulse generator 774 is illustrated. Optionally, the IMD 100 may include multiple pulse generators, similar to pulse generator 774, where each pulse generator is coupled to one or more electrodes and controlled by the microcontroller 764 to deliver select stimulus pulse(s) to the corresponding one or more electrodes.

Microcontroller 764 is illustrated to include timing control circuitry 766 to control the timing of the stimulation pulses (e.g., pacing rate, atrio-ventricular (AV) delay, atrial interconduction (A-A) delay, or ventricular interconduction (V-V) delay, etc.). The timing control circuitry 766 may also be used for the timing of refractory periods, blanking intervals, noise detection windows, evoked response windows, alert periods, marker channel timing, and so on. Microcontroller 764 also has an arrhythmia detector 768 for detecting arrhythmia conditions and a discriminator module 770 to designate series of beats to correspond to be a polymorphic ventricular tachycardia (PVT) or a monomorphic ventricular tachycardia (MVT). The discriminator module 770 may be implemented by one or more processors that implement program instructions, along or in combination with firmware and/or circuitry to carry out the operations described herein. The discriminator module 770 is configured to collect cardiac activity (CA) signals over the first and second sensing channels for a series of beats. The CA signals are electrical signals indicative of changes in an electrical potential along a sensing vector between two or more electrodes. The CA signals are not impedance measurements. In other words, the CA signals are non-impedance electrical CA signals. The discriminator module 770 is further configured to identify a sensed interval delta (SID) from the CA signals for at least a portion of the series of beats, the SID representing a timing interval between i) a COI in the CA signals collected over the first sensing channel and ii) the same COI in the CA signals collected over the second sensing channel. The discriminator module 770 is further configured to designate the series of beats to correspond to be a polymorphic ventricular tachycardia (PVT) or a monomorphic ventricular tachycardia (MVT) based on the SID. Additionally, or alternatively, the discriminator module 770 is further configured to identify an amplitude characteristic of interest (COI) from the CA signals collected over the second sensing channel associated with the bipolar sensing vector for at least a portion of the series of beats, the designation of the series of beats to be PVT or MVT based in part on the COI. Additionally, or alternatively, the discriminator module 770 is further configured to define the COI as an R-wave and the SID to be a timing interval between i) a R-wave in the CA signals collected over the first sensing channel and ii) the same R-wave in the CA signals collected over the second sensing channel.

The microcontroller 764 may further implement a therapy module 772. The therapy module 772 may be further configured to deliver a PVT related therapy or an MVT related therapy based on whether the series of beats are declared to be PVT or MVT. Although not shown, the microcontroller 764 may further include other dedicated circuitry and/or firmware/software components that assist in monitoring various conditions of the patient's heart and managing pacing therapies.

The IMD 100 includes sensing circuitry 780 selectively coupled to one or more electrodes that perform sensing operations, through the switch 792 to detect the presence of cardiac activity in the right chambers of the heart. The sensing circuitry 780 may include dedicated sense amplifiers, multiplexed amplifiers, or shared amplifiers. It may further employ one or more low power, precision amplifiers with programmable gain and/or automatic gain control, bandpass filtering, and threshold detection circuit to selectively sense the cardiac signal of interest. The automatic gain control enables the unit to sense low amplitude signal characteristics of atrial fibrillation. The sensing circuitry 780 is configured to define a first sensing channel coupled to the bipolar electrode combination and configured to define a second sensing channel coupled to the unipolar electrode combination. For example, the sensing circuitry 780 may include the components and circuitry discussed in connection with Figure 2.

Switch 792 determines the sensing polarity of the cardiac signal by selectively closing the appropriate switches. In this way, the clinician may program the sensing polarity independent of the stimulation polarity. The output of the sensing circuitry 780 is connected to the microcontroller 764 which, in turn, triggers or inhibits the pulse generator 774 in response to the absence or presence of cardiac activity. The sensing circuitry 780 receives a control signal 778 from the microcontroller 764 for purposes of controlling the gain, threshold, polarization charge removal circuitry (not shown), and the timing of any blocking circuitry (not shown) coupled to the inputs of the sensing circuitry.

In the example of Figure 7, a single sensing circuit 780 is illustrated. Optionally, the IMD 100 may include multiple sensing circuit, similar to sensing circuit 780, where each sensing circuit is coupled to one or more electrodes and controlled by the microcontroller 764 to sense electrical activity detected at the corresponding one or more electrodes. The sensing circuit 780 is configured to operate at least one channel in a unipolar sensing configuration and at least one channel in a bipolar sensing configuration. The IMD 100 further includes an analog-to-digital (A/D) data acquisition system (DAS) 790 coupled to one or more electrodes via the switch 792 to sample cardiac signals across any pair of desired electrodes. The data acquisition system 790 is configured to acquire intracardiac electrogram signals, convert the raw analog data into digital data, and store the digital data for later processing and/or telemetric transmission to an external device 104 (e.g., a programmer, local transceiver, or a diagnostic system analyzer). The data acquisition system 790 is controlled by a control signal 788 from the microcontroller 764.

The microcontroller 764 is coupled to a memory 752 by a suitable data/address bus 762. The programmable operating parameters used by the microcontroller 764 are stored in memory 752 and used to customize the operation of the IMD 100 to suit the needs of a particular patient. Such operating parameters define, for example, pacing pulse amplitude, pulse duration, electrode polarity, rate, sensitivity, automatic features, arrhythmia detection criteria, and the amplitude, waveshape and vector of each shocking pulse to be delivered to the patient's heart within each respective tier of therapy. The operating parameters of the IMD 100 may be non-invasively programmed into the memory 752 through a telemetry circuit 754 in telemetric communication via communication link 750 with the external device 104. The telemetry circuit 754 allows intracardiac electrograms and status information relating to the operation of the IMD 100 (as contained in the microcontroller 764 or memory 752) to be sent to the external device 104 through the established communication link 750.

The IMD 100 can further include one or more physiologic sensors 756. Such sensors are commonly referred to as "rate-responsive" sensors because they are typically used to adjust pacing stimulation rates according to the exercise state of the patient. However, the physiological sensor 756 may further be used to detect changes in cardiac output, changes in the physiological condition of the heart, or diurnal changes in activity (e.g., detecting sleep and wake states). Signals generated by the physiological sensors 756 are passed to the microcontroller 764 for analysis. The microcontroller 764 responds by adjusting the various pacing parameters (such as rate, AV Delay, V-V Delay, etc.) at which the atrial and ventricular pacing pulses are administered. While shown as being included within the unit 100, the physiologic sensor(s) 756 may be external to the unit 100, yet still be implanted within or carried by the patient. Examples of physiologic sensors might include sensors that, for example, sense respiration rate, pH of blood, ventricular gradient, activity, position/posture, minute ventilation (MV), and so forth.

A battery 758 provides operating power to all of the components in the IMD 100. The battery 758 is capable of operating at low current drains for long periods of time and is capable of providing high-current pulses (for capacitor charging) when the patient requires a shock pulse. The battery 758 also desirably has a predictable discharge characteristic so that elective replacement time can be detected. As one example, the unit 100 employs lithium/silver vanadium oxide batteries. The IMD 100 further includes an impedance measuring circuit 760, which can be used for many things, including: lead impedance surveillance during the acute and chronic phases for proper lead positioning or dislodgement; detecting operable electrodes and automatically switching to an operable pair if dislodgement occurs; measuring respiration or minute ventilation; measuring thoracic impedance for determining shock thresholds; detecting when the device has been implanted; measuring stroke volume; and detecting the opening of heart valves; and so forth. The impedance measuring circuit 760 is coupled to the switch 792 so that any desired electrode may be used.

As explained herein, the IMD 100 can be operated as an implantable cardioverter/defibrillator (ICD) device, which detects the occurrence of an arrhythmia and automatically applies an appropriate electrical shock therapy to the heart aimed at terminating the detected arrhythmia. The therapy module 772 delivers a PVT related therapy or an MVT related therapy based on whether the series of beats are declared to be PVT or MVT. To this end, the microcontroller 764 further controls a shocking circuit 784 by way of a control signal 786. The shocking circuit 780 generates shocking pulses of low (e.g., up to 0.5 joules), moderate (e.g., 0.5-10 joules), or high energy (e.g., 11 to 40 joules), as controlled by the microcontroller 764. Such shocking pulses are applied to the patient's heart through shocking electrodes. It is noted that the shock therapy circuitry is optional and may not be implemented in the IMD, as the various slave pacing units described below will typically not be configured to deliver high voltage shock pulses. On the other hand, it should be recognized that the slave pacing unit can be used within a system that includes backup shock capabilities, and hence such shock therapy circuitry may be included in the IMD.

For example, based on the classification of MVT or PVT, the system and method may take different actions depending upon the type of IMD. For example, the IMD may represent a transvenous or subcutaneous IMD that is configured to treat MVT and PVT. The treatment may include one or more HV shocks, one or more MV shocks, ATP therapy and the like. Additionally or alternatively, the IMD may withhold treatment for a period of time and allow a MVT to self-terminate. If the MVT does not self-terminate, the IMD may then deliver one or more types of therapy. For example, a PVT may warrant a HV shock, while a MVT may be treated with ATP, a MV shock and/or afforded a period of time to self-terminate.

### Closing Statements

It should be clearly understood that the various arrangements and processes broadly described and illustrated with respect to the Figures, and/or one or more individual components or elements of such arrangements and/or one or more process operations associated of such processes, can be employed independently from or together with one or more other components, elements and/or process operations described and illustrated herein. Accordingly, while various arrangements and processes are broadly contemplated, described and illustrated herein, it should be understood that they are provided merely in illustrative and non-restrictive fashion, and furthermore can be regarded as but mere examples of possible working environments in which one or more arrangements or processes may function or operate.

As will be appreciated by one skilled in the art, various aspects may be embodied as a system, method or computer (device) program product. Accordingly, aspects may take the form of an entirely hardware embodiment or an embodiment including hardware and software that may all generally be referred to herein as a "circuit," "module" or "system." Furthermore, aspects may take the form of a computer (device) program product embodied in one or more computer (device) readable storage medium(s) having computer (device) readable program code embodied thereon.

Any combination of one or more non-signal computer (device) readable medium(s) may be utilized. The non-signal medium may be a storage medium. A storage medium may be, for example, an electronic, magnetic, optical, electromagnetic, infrared, or semiconductor system, apparatus, or device, or any suitable combination of the foregoing. More specific examples of a storage medium would include the following: a portable computer diskette, a hard disk, a random access memory (RAM), a dynamic random access memory (DRAM), a read-only memory (ROM), an erasable programmable read-only memory (EPROM or Flash memory), a portable compact disc read-only memory (CD-ROM), an optical storage device, a magnetic storage device, or any suitable combination of the foregoing.

Program code for carrying out operations may be written in any combination of one or more programming languages. The program code may execute entirely on a single device, partly on a single device, as a stand-alone software package, partly on single device and partly on another device, or entirely on the other device. In some cases, the devices may be connected through any type of network, including a local area network (LAN) or a wide area network (WAN), or the connection may be made through other devices (for example, through the Internet using an Internet Service Provider) or through a hard wire connection, such as over a USB connection. For example, a server having a first processor, a network interface, and a storage device for storing code may store the program code for carrying out the operations and provide this code through its network interface via a network to a second device having a second processor for execution of the code on the second device.

Aspects are described herein with reference to the figures, which illustrate example methods, devices and program products according to various example embodiments. These program instructions may be provided to a processor of a general purpose computer, special purpose computer, or other programmable data processing device or information handling device to produce a machine, such that the instructions, which execute via a processor of the device implement the functions/acts specified. The program instructions may also be stored in a device readable medium that can direct a device to function in a particular manner, such that the instructions stored in the device readable medium produce an article of manufacture including instructions which implement the function/act specified. The program instructions may also be loaded onto a device to cause a series of operational steps to be performed on the device to produce a device implemented process such that the instructions which execute on the device provide processes for implementing the functions/acts specified.

The units/modules/applications herein may include any processor-based or microprocessor-based system including systems using microcontrollers, reduced instruction set computers (RISC), application specific integrated circuits (ASICs), field-programmable gate arrays (FPGAs), logic circuits, and any other circuit or processor capable of executing the functions described herein. Additionally or alternatively, the modules/controllers herein may represent circuit modules that may be implemented as hardware with associated instructions (for example, software stored on a tangible and non-transitory computer readable storage medium, such as a computer hard drive, ROM, RAM, or the like) that perform the operations described herein. The above examples are exemplary only, and are thus not intended to limit in any way the definition and/or meaning of the term "controller." The units/modules/applications herein may execute a set of instructions that are stored in one or more storage elements, in order to process data. The storage elements may also store data or other information as desired or needed. The storage element may be in the form of an information source or a physical memory element within the modules/controllers herein. The set of instructions may include various commands that instruct the modules/applications herein to perform specific operations such as the methods and processes of the various embodiments of the subject matter described herein. The set of instructions may be in the form of a software program. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to user commands, or in response to results of previous processing, or in response to a request made by another processing machine.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

It is to be understood that the above description is intended to be illustrative, and not restrictive. For example, the above-described embodiments (and/or aspects thereof) may be used in combination with each other. In addition, many modifications may be made to adapt a particular situation or material to the teachings herein without departing from its scope. While the dimensions, types of materials and coatings described herein are intended to define various parameters, they are by no means limiting and are illustrative in nature. Many other embodiments will be apparent to those of skill in the art upon reviewing the above description. The scope of the embodiments should, therefore, be determined with reference to the appended claims. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects or order of execution on their acts.

## Claims

1. An implantable medical device, IMD, (100), comprising:
electrodes (101, 122, 124, 126, 132-138) configured to be located in or about a heart, the electrodes (101, 122, 124, 126, 132-138) including a bipolar electrode combination (122, 124, 126, 132-138) that defines a bipolar sensing vector, the electrodes (101, 122, 124, 126, 132-138) including a unipolar electrode combination (101, 122, 124, 126, 132-138) that defines a unipolar sensing vector;
sensing circuitry (204, 780) configured to define a first sensing channel (208) coupled to the bipolar electrode combination (122, 124, 126, 132-138) and configured to define a second sensing channel (210) coupled to the unipolar electrode combination (101, 122, 124, 126, 132-138);
memory (752) to store program instructions;
one or more processors (764) configured to implement the program instructions to:
collect cardiac activity, CA, signals over the first and second sensing channels (208, 210) for a series of beats; and
identify a sensed interval delta, SID, from the CA signals for at least a portion of the series of beats, the SID representing a timing interval between i) a characteristic of interest, COI, in the CA signals collected over the first sensing channel (208) and ii) the same COI in the CA signals collected over the second sensing channel (210), **characterized in that** the one or more processors (764) are further configured to implement the program instructions to:
designate the series of beats to correspond to be a polymorphic ventricular tachycardia, PVT, or a monomorphic ventricular tachycardia, MVT, based on the SID.

2. The IMD of claim 1, wherein the one or more processors (764) are further configured to deliver a PVT related therapy or an MVT related therapy based on whether the series of beats are declared to be PVT or MVT.

3. The IMD of claim 1 or 2, wherein the one or more processors (764) are further configured to identify an amplitude characteristic of interest, COI, from the CA signals collected over the second sensing channel (210) associated with the unipolar sensing vector for at least a portion of the series of beats, the designation of the series of beats to be PVT or MVT based in part on the COI.

4. The IMD of claim 3, wherein the COI is an R-wave and the SID represents the timing interval between i) a R-wave in the CA signals collected over the first sensing channel (208) and ii) the same R-wave in the CA signals collected over the second sensing channel (210).

5. The IMD of claim 3 or 4, wherein the one or more processors (764) are further configured to compare the SID and the amplitude COI to corresponding SID and COI criteria and based on the comparisons, the one or more processors (752) designate the current series of beats to be a PVT or MVT.

6. The IMD of claim 5, wherein the SID and COI criteria include SID and COI thresholds, respectively, and wherein the one or more processors (764) are further configured to at least one of:
a) designate the series of beats to be PVT when the SID and amplitude COI exceed the corresponding SID and COI thresholds;
b) designate the series of beats to be MVT when the SID and amplitude COI do not exceed the corresponding SID and COI thresholds; or
c) designate a discrimination not possible condition when only one of the SID and amplitude COI exceed the corresponding SID and COI thresholds and another of the SID and amplitude COI do not exceed the corresponding SID and COI thresholds.

7. The IMD of any one of claims 1 to 6, wherein the first sensing channel (208) includes a first amplifier (212) and a first digital processing circuit (214), the first amplifier (212) includes input terminals that are coupled to the bipolar electrode combination (122, 124, 126, 132-138), the second sensing channel (210) including a second amplifier (216) and a second digital processing circuit (218), the second amplifier (216) including input terminals that are coupled to the unipolar electrode combination (101, 122, 124, 126, 132-138).

8. The IMD of any one of claims 1 to 7, wherein the bipolar and unipolar electrode combinations (101, 122, 124, 126, 132-138) include at least one common electrode (122, 124, 126, 132-138).

9. The IMD of any one of claims 1 ot 8, wherein the bipolar electrode combination (122, 124, 126, 132-138) includes first and second electrodes (122, 124, 126, 132-138) located within or proximate to one or more chambers of the heart, and the unipolar electrode combination (101, 122, 124, 126, 132-138) includes a third electrode (101) located remote from the heart.

10. The IMD of any one of claims 1 to 9, wherein the one or more processors (764) are configured to store the designation of the PVT or MVT, the IMD (752) further comprising a telemetry circuitry (754) configured to downloaded the designation of the PVT or MVT, from the IMD (100), to an external device (104).

11. A method, comprising:
identifying with the one or more processors of the IMD of claim 1 a SID from CA signals collected over a first sensing channel (208) coupled to a bipolar electrode combination (122, 124, 126, 132-138) and a second sensing channel (210) coupled to a unipolar electrode combination (101, 122, 124, 126, 132-138) for at least a portion of a series of beats, the SID representing a timing interval between i) a COI in the CA signals collected over the first sensing channel (208) and ii) the same COI in the CA signals collected over the second sensing channel (201), **characterized by**:
designating with the one or more processors the series of beats to correspond to be a PVT or a MVT based on the SID.

12. The method of claim 11, further comprising identifying with the one or more processors an amplitude characteristic of interest, COI, from the CA signals collected over the second sensing channel (210) associated with the bipolar sensing vector for at least a portion of the series of beats, the designating of the series of beats to be PVT or MVT based in part on the COI.

13. The method of claim 12, wherein the COI is an R-wave and the SID represents the timing interval between i) a R-wave in the CA signals collected over the first sensing channel and ii) the same R-wave in the CA signals collected over the second sensing channel.

14. The method of claim 12 or 13, further comprising comparing with the one or more processors the SID and the amplitude COI to corresponding SID and COI criteria and based on the comparisons, designating with the one or more processors the current series of beats to be a PVT or MVT.

15. The method of claim 14, wherein the SID and COI criteria include SID and COI thresholds, respectively, and wherein the method further comprises designating with the one or more processors the series of beats to be PVT when the SID and amplitude COI exceed the corresponding SID and COI thresholds.

16. The method of claim 14 or 15, wherein the SID and COI criteria include SID and COI thresholds, respectively, and wherein the method further comprises designating with the one or more processors the series of beats to be MVT when the SID and amplitude COI do not exceed the corresponding SID and COI thresholds.

17. The method of any one of claims 14 to 16, wherein the SID and COI criteria include SID and COI thresholds, respectively, and wherein the method further comprises designating with the one or more processors a discrimination not possible condition when only one of the SID and amplitude COI exceed the corresponding SID and COI thresholds and another of the SID and amplitude COI do not exceed the corresponding SID and COI thresholds.

18. The method of any one of claims 11 to 17, wherein the first and second sensing channels (208, 210) utilize at least one common electrode (122, 124, 126, 132-138).

## Patentansprüche

1. Implantierbare medizinische Vorrichtung, IMD, (100), die umfasst:
Elektroden (101, 122, 124, 126, 132-138), die konfiguriert sind, um in einem Herzen oder um ein Herz herum angeordnet zu werden, wobei die Elektroden (101, 122, 124, 126, 132-138) eine bipolare Elektrodenkombination (122, 124, 126, 132-138) beinhalten, die einen bipolaren Erfassungsvektor definiert, wobei die Elektroden (101, 122, 124, 126, 132-138) eine unipolare Elektrodenkombination (101, 122, 124, 126, 132-138) beinhaltet, die einen unipolaren Erfassungsvektor definiert;
einen Erfassungsschaltkreis (204, 780), der konfiguriert ist, um einen ersten Erfassungskanal (208) zu definieren, der mit der bipolaren Elektrodenkombination (122, 124, 126, 132-138) gekoppelt ist, und dazu konfiguriert ist, einen zweiten Erfassungskanal (210) zu definieren, der mit der unipolaren Elektrodenkombination (101, 122, 124, 126, 132-138) gekoppelt ist;
einen Speicher (752), um Programmanweisungen zu speichern;
einen oder mehrere Prozessoren (764), die konfiguriert sind, um die Programmanweisungen umzusetzen zum:
Sammeln von Signalen einer Herzaktivität, CA, über den ersten und den zweiten Erfassungskanal (208, 210) für eine Reihe von Schlägen; und
Identifizieren eines erfassten Intervalls Delta, SID, anhand der CA-Signale für zumindest einen Teil der Reihe von Schlägen, wobei das SID ein Zeitintervall zwischen i) einem Charakteristikum von Interesse, COI, in den CA-Signalen, die über den ersten Erfassungskanal (208) gesammelt werden, und ii) dem gleichen COI in den CA-Signalen, die über den zweiten Erfassungskanal (210) gesammelt werden, darstellt, **dadurch gekennzeichnet, dass** der eine oder die mehreren Prozessoren (764) ferner konfiguriert sind, um die Programmanweisungen umzusetzen zum:
Bestimmen, dass die Reihe von Schlägen einer polymorphen ventrikulären Tachykardie, PVT, oder einer monomorphen ventrikulären Tachykardie, MVT, entspricht, basierend auf dem SID.

2. IMD nach Anspruch 1, wobei der eine oder die mehreren Prozessoren (764) ferner konfiguriert sind, um basierend darauf, ob die Reihe von Schlägen als PVT oder MVT ausgewiesen ist, eine PVT-bezogene Therapie oder eine MVT-bezogene Therapie zu verabreichen.

3. IMD nach Anspruch 1 oder 2, wobei der eine oder die mehreren Prozessoren (764) ferner konfiguriert sind, um ein Charakteristikum von Interesse, COI, in Bezug auf eine Amplitude anhand der CA-Signale zu identifizieren, die über den zweiten Erfassungskanal (210) gesammelt werden, der dem unipolaren Erfassungsvektor zugeordnet ist, für zumindest einen Teil der Reihe von Schlägen, wobei die Reihe von Schlägen teilweise basierend auf dem COI als PVT oder MVT bestimmt wird.

4. IMD nach Anspruch 3, wobei das COI eine R-Zacke ist und das SID das Zeitintervall zwischen i) einer R-Zacke in den CA-Signalen, die über den ersten Erfassungskanal (208) gesammelt werden, und ii) der gleichen R-Zacke in den CA-Signalen, die über den zweiten Erfassungskanal (210) gesammelt werden, darstellt.

5. IMD nach Anspruch 3 oder 4, wobei der eine oder die mehreren Prozessoren (764) ferner konfiguriert sind, um das SID und den Amplituden-COI mit entsprechenden SID- und COI-Kriterien zu vergleichen und wobei der eine oder die mehreren Prozessoren (752) die aktuelle Reihe von Schlägen basierend auf den Vergleichen als PVT oder MVT bestimmen.

6. IMD nach Anspruch 5, wobei die SID- und COI-Kriterien SID- bzw. COI-Schwellenwerte beinhalten und wobei der eine oder die mehreren Prozessoren (764) ferner zumindest zu einem konfiguriert sind aus:
**a)** Bestimmen der Reihe von Schlägen als PVT, wenn SID und Amplituden-COI die entsprechenden SID- und COI-Schwellenwerte überschreiten;
**b)** Bestimmen der Reihe von Schlägen als MVT, wenn SID und Amplituden-COI die entsprechenden SID- und COI-Schwellenwerte nicht überschreiten; oder
**c)** Bestimmen eines Unterscheidung-nicht-möglich-Zustands, wenn nur eines des SID und des Amplituden-COI die entsprechenden SID- und COI-Schwellenwerte überschreitet und ein anderes von SID und Amplituden-COI die entsprechenden SID- und COI-Schwellenwerte nicht überschreitet.

7. IMD nach einem der Ansprüche 1 bis 6, wobei der erste Erfassungskanal (208) einen ersten Verstärker (212) und eine erste digitale Verarbeitungsschaltung (214) beinhaltet, der erste Verstärker (212) Eingangsklemmen beinhaltet, die mit der bipolaren Elektrodenkombination (122, 124 124, 126, 132-138) gekoppelt sind, wobei der zweite Erfassungskanal (210) einen zweiten Verstärker (216) und eine zweite digitale Verarbeitungsschaltung (218) beinhaltet, wobei der zweite Verstärker (216) Eingangsklemmen, beinhaltet, die mit der unipolaren Elektrodenkombination (101, 122, 124, 126, 132-138) gekoppelt sind.

8. IMD nach einem der Ansprüche 1 bis 7, wobei die bipolare und die unipolare Elektrodenkombination (101, 122, 124, 126, 132-138) zumindest eine gemeinsame Elektrode (122, 124, 126, 132-138) beinhalten.

9. IMD nach einem der Ansprüche 1 bis 8, wobei die bipolare Elektrodenkombination (122, 124, 126, 132-138) erste und zweite Elektroden (122, 124, 126, 132-138) beinhaltet, die sich innerhalb oder in der Nähe einer oder mehrerer Kammern des Herzens befinden, und die unipolare Elektrodenkombination (101, 122, 124, 126, 132-138) eine dritte Elektrode (101) beinhaltet, die sich abseits des Herzens befindet.

10. IMD nach einem der Ansprüche 1 bis 9, wobei der eine oder die mehreren Prozessoren (764) konfiguriert sind, um die Bestimmung der PVT oder MVT zu speichern, wobei die IMD (752) ferner eine Telemetrieschaltung (754) umfasst, die konfiguriert ist, um die Bestimmung der PVT oder MVT von der IMD (100) auf ein externes Gerät (104) herunterzuladen.

11. Verfahren, das umfasst:
Identifizieren eines SID anhand von CA-Signalen, die über einen ersten Erfassungskanal (208), der mit einer bipolaren Elektrodenkombination (122, 124, 126, 132-138) gekoppelt ist, und einen zweiten Erfassungskanal (210), der mit einer unipolaren Elektrodenkombination (101, 122, 124, 126, 132-138) gekoppelt ist, gesammelt werden, für zumindest einen Teil einer Reihe von Schlägen durch den einen oder die mehreren Prozessoren des IMD nach Anspruch 1, wobei das SID ein Zeitintervall zwischen i) einem COI in den CA-Signalen, die über den ersten Erfassungskanal (208) gesammelt werden, und ii) dem gleichen SOI in den CA-Signalen, die über den zweiten Erfassungskanal (201) gesammelt werden, darstellt, **gekennzeichnet durch**:
Bestimmen durch den einen oder die mehreren Prozessoren, dass die Reihe von Schlägen einer PVT oder einer MVT entspricht, basierend auf dem SID.

12. Verfahren nach Anspruch 11, das ferner das Identifizieren eines Charakteristikums von Interesse, COI, in Bezug auf eine Amplitude anhand der CA-Signale, die über den zweiten Erfassungskanal (210) gesammelt werden, der dem bipolaren Erfassungsvektor zugeordnet ist, für zumindest einen Teil der Reihe von Schlägen, durch den einen oder die mehreren Prozessoren umfasst, wobei die Reihe von Schlägen teilweise basierend auf dem COI als PVT oder MVT bestimmt wird.

13. Verfahren nach Anspruch 12, wobei das COI eine R-Zacke ist und das SID das Zeitintervall zwischen i) einer R-Zacke in den CA-Signalen, die über den ersten Erfassungskanal gesammelt werden, und ii) der gleichen R-Zacke in den CA-Signalen, die über den zweiten Erfassungskanal gesammelt werden, darstellt.

14. Verfahren nach Anspruch 12 oder 13, das ferner das Vergleichen des SID und des Amplituden-COI mit entsprechenden SID- und COI-Kriterien durch den einen oder die mehreren Prozessoren und das Bestimmen, dass die aktuelle Reihe von Schlägen eine PVT oder MVT ist, basierend auf den Vergleichen durch den einen oder die mehreren Prozessoren umfasst.

15. Verfahren nach Anspruch 14, wobei die SID- und COI-Kriterien SID- bzw. COI-Schwellenwerte beinhalten und wobei das Verfahren ferner das Bestimmen, dass die Reihe von Schlägen eine PVT ist, wenn das SID und das Amplituden-COI die entsprechenden SID- und COI-Schwellenwerte überschreiten, durch den einen oder die mehreren Prozessoren umfasst.

16. Verfahren nach Anspruch 14 oder 15, wobei die SID- und COI-Kriterien SID- bzw. COI-Schwellenwerte beinhalten und wobei das Verfahren ferner das Bestimmen, dass die Reihe von Schlägen eine MVT ist, wenn das SID und das Amplituden-COI die entsprechenden SID- und COI-Schwellenwerte nicht überschreiten, durch den einen oder die mehreren Prozessoren umfasst.

17. Verfahren nach einem der Ansprüche 14 bis 16, wobei die SID- und COI-Kriterien SID- bzw. COI-Schwellenwerte beinhalten und wobei das Verfahren ferner das Bestimmen eines Unterscheidung-nicht-möglich-Zustands durch den einen oder die mehreren Prozessoren umfasst, wenn nur eines des SID und des Amplituden-COI die entsprechenden SID- und COI-Schwellenwerte überschreitet und ein anderes von SID und Amplituden-COI die entsprechenden SID- und COI-Schwellenwerte nicht überschreitet.

18. Verfahren nach einem der Ansprüche 11 bis 17, wobei der erste und der zweite Erfassungskanal (208, 210) zumindest eine gemeinsame Elektrode (122, 124, 126, 132-138) verwenden.

## Revendications

1. Dispositif médical implantable, IMD (100), comprenant :
des électrodes (101, 122, 124, 126, 132-138) configurées pour être situées dans ou autour d'un cœur, les électrodes (101, 122, 124, 126, 132-138) comportant une combinaison d'électrodes bipolaires (122, 124, 126, 132-138) qui définit un vecteur de détection bipolaire, les électrodes (101, 122, 124, 126, 132-138) comportant une combinaison d'électrodes unipolaires (101, 122, 124, 126, 132-138) qui définit un vecteur de détection unipolaire ;
un circuit de détection (204, 780) configuré pour définir un premier canal de détection (208) couplé à la combinaison d'électrodes bipolaires (122, 124, 126, 132-138) et configuré pour définir un second canal de détection (210) couplé à la combinaison d'électrodes unipolaires (101, 122, 124, 126, 132-138) ;
une mémoire (752) pour stocker les instructions de programme ;
un ou plusieurs processeurs (764) configurés pour mettre en œuvre les instructions du programme pour :
collecter des signaux d'activité cardiaque, CA, sur les premier et second canaux de détection (208, 210) pour une série de battements ; et
identifier un delta d'intervalle détecté, SID, à partir des signaux CA pour au moins une partie de la série de battements, le SID représentant un intervalle de temps entre i) une caractéristique d'intérêt, COI, dans les signaux CA collectés sur le premier canal de détection (208) et ii) la même COI dans les signaux CA collectés sur le second canal de détection (210), **caractérisé en ce que** les un ou plusieurs processeurs (764) sont en outre configurés pour mettre en œuvre les instructions de programme pour :
désigner la série de battements devant correspondre à une tachycardie ventriculaire polymorphe, PVT, ou à une tachycardie ventriculaire monomorphe, MVT, en fonction du SID.

2. IMD selon la revendication 1, dans lequel les un ou plusieurs processeurs (764) sont en outre configurés pour délivrer une thérapie liée au PVT ou une thérapie liée au MVT selon que la série de battements est déclarée comme étant du PVT ou du MVT.

3. IMD selon la revendication 1 ou 2, dans lequel les un ou plusieurs processeurs (764) sont en outre configurés pour identifier une caractéristique d'amplitude d'intérêt, COI, à partir des signaux CA collectés sur le second canal de détection (210) associé au vecteur de détection unipolaire pour au moins une partie de la série de battements, la désignation de la série de battements devant être PVT ou MVT sur la base en partie de la COI.

4. IMD selon la revendication 3, dans lequel la COI est une onde R et le SID représente l'intervalle de temps entre i) une onde R dans les signaux CA collectés sur le premier canal de détection (208) et ii) la même onde R dans les signaux CA collectés sur le second canal de détection (210).

5. IMD selon la revendication 3 ou 4, dans lequel les un ou plusieurs processeurs (764) sont en outre configurés pour comparer le SID et la COI d'amplitude aux critères SID et COI correspondants et sur la base des comparaisons, les un ou plusieurs processeurs (752) désignent la série actuelle de battements comme étant un PVT ou un MVT.

6. IMD selon la revendication 5, dans lequel les critères SID et COI comportent respectivement des seuils SID et COI, et dans lequel les un ou plusieurs processeurs (764) sont en outre configurés pour au moins l'une des actions suivantes :
a) désigner la série de battements comme étant PVT lorsque le SID et la COI d'amplitude dépassent les seuils SID et COI correspondants ;
b) désigner la série de battements comme étant MVT lorsque le SID et la COI d'amplitude ne dépassent pas les seuils SID et COI correspondants ; ou
c) désigner une condition de discrimination impossible lorsque seulement l'un des SID et de la COI d'amplitude dépasse les seuils SID et COI correspondants et l'autre des SID et de la COI d'amplitude ne dépasse pas les seuils SID et COI correspondants.

7. IMD selon l'une quelconque des revendications 1 à 6, dans lequel le premier canal de détection (208) comporte un premier amplificateur (212) et un premier circuit de traitement numérique (214), le premier amplificateur (212) comporte des bornes d'entrée qui sont couplées à la combinaison d'électrodes bipolaires (122, 124, 126, 132-138), le second canal de détection (210) comportant un second amplificateur (216) et un second circuit de traitement numérique (218), le second amplificateur (216) comportant des bornes d'entrée qui sont couplées à la combinaison d'électrodes unipolaires (101, 122, 124, 126, 132-138).

8. IMD selon l'une quelconque des revendications 1 à 7, dans lequel les combinaisons d'électrodes bipolaires et unipolaires (101, 122, 124, 126, 132-138) comportent au moins une électrode commune (122, 124, 126, 132-138).

9. IMD selon l'une quelconque des revendications 1 ou 8, dans lequel la combinaison d'électrodes bipolaires (122, 124, 126, 132-138) comporte des première et deuxième électrodes (122, 124, 126, 132-138) situées à l'intérieur ou à proximité d'une ou de plusieurs chambres du cœur, et la combinaison d'électrodes unipolaires (101, 122, 124, 126, 132-138) comporte une troisième électrode (101) située à distance du cœur.

10. IMD selon l'une quelconque des revendications 1 à 9, dans lequel les un ou plusieurs processeurs (764) sont configurés pour stocker la désignation du PVT ou du MVT, l'IMD (752) comprenant en outre un circuit de télémétrie (754) configuré pour télécharger la désignation du PVT ou du MVT, de l'IMD (100), vers un dispositif externe (104).

11. Procédé, comprenant :
l'identification, avec un ou plusieurs processeurs de l'IMD selon la revendication 1, d'un SID à partir de signaux CA collectés sur un premier canal de détection (208) couplé à une combinaison d'électrodes bipolaires (122, 124, 126, 132-138) et un second canal de détection (210) couplé à une combinaison d'électrodes unipolaires (101, 122, 124, 126, 132-138) pour au moins une partie d'une série de battements, le SID représentant un intervalle de temps entre i) une COI dans les signaux CA collectés sur le premier canal de détection (208) et ii) la même COI dans les signaux CA collectés sur le second canal de détection (201), **caractérisé par** :
la désignation, avec les un ou plusieurs processeurs, de la série de battements à correspondre comme étant un PVT ou un MVT sur la base du SID.

12. Procédé selon la revendication 11, comprenant en outre l'identification avec les un ou plusieurs processeurs d'une caractéristique d'amplitude d'intérêt, COI, à partir des signaux CA collectés sur le second canal de détection (210) associé au vecteur de détection bipolaire pour au moins une partie de la série de battements, la désignation de la série de battements comme étant PVT ou MVT étant basée en partie sur la COI.

13. Procédé selon la revendication 12, dans lequel la COI est une onde R et le SID représente l'intervalle de temps entre i) une onde R dans les signaux CA collectés sur le premier canal de détection et ii) la même onde R dans les signaux CA collectés sur le second canal de détection.

14. Procédé selon la revendication 12 ou 13, comprenant en outre la comparaison avec les un ou plusieurs processeurs du SID et de la COI d'amplitude aux critères SID et COI correspondants et sur la base des comparaisons, la désignation avec les un ou plusieurs processeurs de la série actuelle de battements comme étant un PVT ou un MVT.

15. Procédé selon la revendication 14, dans lequel les critères SID et COI comportent respectivement des seuils SID et COI, et dans lequel le procédé comprend en outre la désignation, avec les un ou plusieurs processeurs, de la série de battements à PVT lorsque le SID et la COI d'amplitude dépassent les seuils SID et COI correspondants.

16. Procédé selon la revendication 14 ou 15, dans lequel les critères SID et COI comportent respectivement des seuils SID et COI, et dans lequel le procédé comprend en outre la désignation, avec les un ou plusieurs processeurs, de la série de battements à MVT lorsque le SID et la COI d'amplitude ne dépassent pas les seuils SID et COI correspondants.

17. Procédé selon l'une quelconque des revendications 14 à 16, dans lequel les critères SID et COI comportent respectivement des seuils SID et COI, et dans lequel le procédé comprend en outre la désignation avec les un ou plusieurs processeurs d'une condition non possible de discrimination lorsque seulement l'un des critères SID et COI d'amplitude dépasse les seuils SID et COI correspondants et l'autre des SID et COI d'amplitude ne dépasse pas les seuils SID et COI correspondants.

18. Procédé selon l'une quelconque des revendications 11 à 17, dans lequel les premier et second canaux de détection (208, 210) utilisent au moins une électrode commune (122, 124, 126, 132-138).
